# EUROPÄISCHE PATENTSCHRIFT

(11) **EP 0 586 972 B1**
(45) Veröffentlichungstag und Bekanntmachung des Hinweises auf die Patenterteilung: **28.04.1999**
(21) Anmeldenummer: 93113629.5
(22) Anmeldetag: 26.08.1993
(51) Int. Cl.: A61B 1/24, A61B 13/00, A61B 1/267

(54) **Laryngoskopspatel**
Laryngoscope blade
Spatule de laryngoscope

(30) Priorität: 05.09.1992 DE 4229739; 23.12.1992 DE 4243790
(43) Veröffentlichungstag der Anmeldung: 16.03.1994
(73) Patentinhaber: Karl Storz GmbH & Co., 78532 Tuttlingen (DE)
(72) Erfinder: Storz, Karl, Dr. med. h.c., (DE)
(74) Vertreter: Weller, Wolfgang, Dr.rer.nat.

(56) Entgegenhaltungen:
- EP-A- 0 184 588
- EP-A- 0 339 541
- DE-U- 8 526 662
- US-A- 4 565 187
- US-A- 5 060 633

## Beschreibung

Die Erfindung betrifft einen Laryngoskopspatel, insbesondere zum Einfügen von Narkosetuben in die Luftröhre, mit einem Handgriff, mit einem spatelförmigen Teil und mit einem Lichtträger, über den von einer Lichtquelle ausgehende Lichtstrahlen geleitet werden, wobei am spatelförmigen Teil eine Ausnehmung vorgesehen ist, in der zumindest ein Bereich des Lichtträgers lösbar aufnehmbar und dort fixierbar ist, wobei der Lichtträger an seinem der Lichtquelle zugeordneten Ende aufgeweitet ist, und wobei die Ausnehmung am spatelförmigen Teil einen aufgeweiteten Bereich aufweist, in dem der aufgeweitete Bereich des Lichtträgers aufnehmbar ist.

Ein derartiger Laryngoskopspatel ist aus der EP-A-0 184 588 bekannt.

Bei diesem Laryngoskopspatel ist im spatelförmigen Teil eine seitliche Ausnehmung vorgesehen, in die ein als hohler Kunststoffkörper ausgebildeter Lichtträger bzw. Lichtleiter seitlich im Paßsitz eindrückbar ist. Am lichteintrittseitigen Ende ist der Lichtträger aufgeweitet, dazu weist die Aussparung eine entsprechende Aufweitung auf. Aufgrund von Fertigungstoleranzen und aufgrund von Materialermüdung ist es möglich, daß bei der Handhabung der Lichtträger seitlich vom spatelförmigen Teil herausfällt, da keine mechanischen Sperren, mit Ausnahme des Paß- bzw. Preßsitzes, vorgesehen sind.

Aus der EP-A-0 339 541 ist ein Laryngoskopspatel bekannt, bei dem der spatelförmige Teil blattförmig ausgebildet ist, wobei an der Unterseite des Spatelblattes eine etwa mittig verlaufende Längsnut herausgearbeitet ist. In diese Nut ist ein in einem Gehäuse aufgenommener Lichtleiter als Formkörper angepaßt ortsfest einfügbar. Eine Schnappbefestigung sorgt dafür, daß das dicht verschlossene Formgehäuse, das den Lichtleiter enthält, arretierbar und austauschbar im spatelförmigen Teil untergebracht ist.

Aus der US-A-5 060 633 ist ein Laryngoskopspatel bekannt, der mit einem austauschbaren Lichtleiter versehen ist. Der rohrförmige Lichtleiter mit durchgehend gleichem Außendurchmesser wird über eine Feststellschraube am spatelförmigen Teil fixiert.

Aus der US-A-4 958 624 ist ein Laryngoskopspatel bekannt, bei dem der durchgängig rohrförmige Lichtleiter in eine Bohrung im Kupplungskopf einschiebbar ist. Das lichtaustrittseitige Ende ist dabei in einer taschenartigen Ausnehmung eingeschoben. Das Kupplungsteil ist über eine Schraube mit dem Spatelteil verschraubt.

Beiden zuvor genannten Laryngoskopspateln ist gemein, daß zum Auswechseln des Lichtleiters eine Schraube gelöst und entsprechend aufbewahrt werden muß. Nachdem der Lichtleiter ersetzt wurde, muß wieder eine Schraube eingedreht werden.

Aus der DE-U-85 26 622 ist ein Laryngoskopspatel mit einem auswechselbaren Lichtleiter bekannt, der als gebogener rohrförmiger Körper mit durchgehend gleichem Durchmesser ausgebildet ist. Der Lichtleiter kann in eine seitliche Ausnehmung über zumindest einen Bereich des Lichtleiters eingeschoben werden und wird über einen Kugelrastmechanismus gehalten. Dazu ist eine exakt bearbeitete zusätzliche Ausnehmung vorgesehen, in der eine federbelastete Sperrkugel aufgenommen ist, die beim Abnehmen oder Einlegen des Lichtleiters in die zusätzliche Ausnehmung gegen die Kraft der Feder eingedrückt und anschließend durch die Kraft wieder so weit ausgedrückt wird, daß die Kugel ein Herausfallen des Lichtleiters mechanisch sperrt.

Aus der US-A-4 565 187 ist ein Laryngoskopspatel bekannt, bei dem ein flexibler Lichtleiter, dessen lichteintrittsseitiges Ende mit einem Gewinde versehen ist, gelöst bzw. vom Laryngoskopspatel abgedreht wird und anschließend ein neuer Lichtleiter über eine Schraubverbindung eingedreht wird. Dazu wird der Lichtleiter zunächst vom spatelförmigen Teil gelöst, nach Bewerkstelligung der Schraubverbindung wird der flexible Lichtleiter in einem entsprechenden Kanal am spatelförmigen Teil eingeschoben.

Aufgabe der vorliegenden Erfindung ist es, einen Laryngoskopspatel der eingangs genannten Art derart weiterzuentwickeln, daß ein einfaches und schnelles Auswechseln des Lichtträgers möglich ist, wobei der montierte Lichtleiter sicher gehalten ist, wobei dies mit möglichst einfachem konstruktivem Aufwand ermöglicht werden soll.

Erfindungsgemäß wird die Aufgabe dadurch gelöst, daß der Lichtträger Glasfasern enthält und daß die Ausnehmung in dem Bereich, in dem der aufgeweitete Bereich des Lichtträgers aufnehmbar ist, eine schmale seitliche Öffnung aufweist, über die der Lichtträger lediglich in dessen nicht aufgeweiteten Bereich einschiebbar ist und aus dieser ersten Stellung durch eine Verschwenkbewegung in seine Endstellung bringbar ist, wobei in dieser Endstellung der aufgeweitete Bereich des Lichtträgers in den aufgeweiteten Bereich der Aufnehmung eingreift und wegen der schmalen seitlichen Öffnung vor seitlichem Abfallen gesperrt ist.

Diese Maßnahme hat nun den Vorteil, daß der Lichtträger in einem ersten Schritt über seinen durchmessergeringen Bereich seitlich durch die entsprechend durchmessergeringere Öffnung der Ausnehmung eingeschoben werden kann. Anschließend wird das aufgeweitete Ende des Lichtträgers in den aufgeweiteten Bereich der Ausnehmung abgesenkt.

Dieser Vorgang ist einfach und rasch durchzuführen. Der Lichtträger ist dann vor einem seitlichen Herausfallen aus der Ausnehmung mechanisch gesperrt. Der umgekehrte Vorgang, das Abnehmen, ist gleichermaßen einfach durchzuführen.

In einer weiteren Ausgestaltung der Erfindung ist der Lichtträger im Bereich seiner Kupplung mit der Lichtquelle mit einem Rohrflansch versehen.

Diese Maßnahme hat den Vorteil, daß dieser endseitige Rohrflansch als Anschlag für die Eintauch- bzw. Einsenkbewegung dient.

In einer weiteren Ausgestaltung der Erfindung ist der Lichtträger im aufgeweiteten Bereich mit einer Gewinde- bzw. Bajonettführung versehen.

Diese Maßnahme hat nun den Vorteil, daß über die Gewinde- oder Bajonettführung die Verschwenk- und Einsenkbewegung in die Aufweitung zielgerichtet und vorgegeben abläuft, so daß der Vorgang besonders einfach und zu einem exakten Paßsitz führend durchgeführt werden kann.

In einer weiteren Ausgestaltung der Erfindung ist das spatelförmige Teil mit einer Öffnung versehen, durch die das lichtaustrittsseitige Ende des Lichtträgers hindurch geht.

Eine verstärkte Fixierung kann dadurch erreicht werden, daß der Lichtträger zusätzlich noch über eine Magnetwirkung fixiert ist.

Weitere Vorteile und Einzelheiten der Erfindung ergeben sich aus der folgenden Beschreibung eines Ausführungsbeispiels im Zusammenhang mit den beiliegenden Zeichnungen.

Es zeigen:
- Fig. 1: eine Seitenansicht eines erfindungsgemäßen Laryngoskopspatels,
- Fig. 2: eine perspektivische Darstellung des spatelförmigen Teils mit ausgeschwenktem Lichtträger, und
- Fig. 3: eine weitere perspektivische Darstellung mit dem Lichtträger in seiner Endstellung.

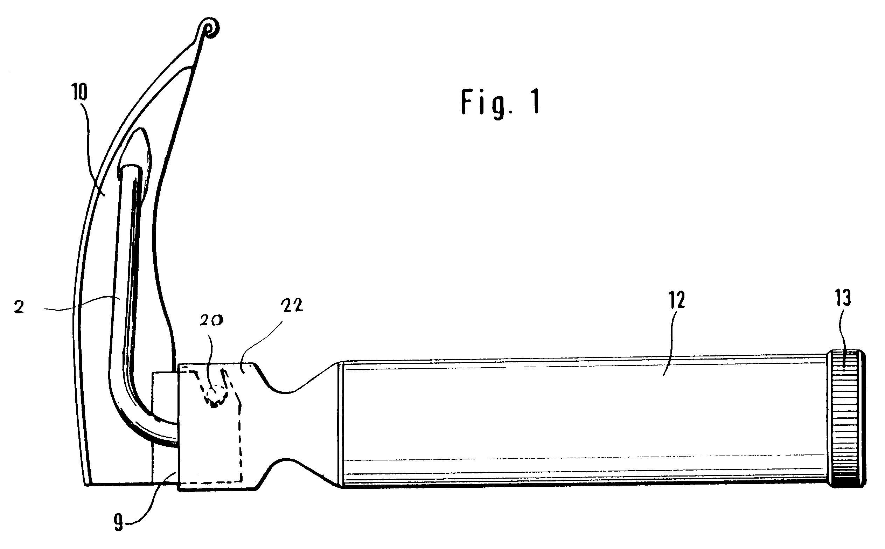

Ein in Fig. 1 dargestellter Laryngoskopspatel weist einen Handgriff 12 auf, der endseitig mit einer Schraubkappe 13 verschlossen ist.

Im Handgriff 12 ist in an sich bekannter Weise eine Batterie sowie eine Miniaturglühlampe angeordnet, und zwar an Glasfasern angrenzend, die in einen Lichtträger 2 führen. Der Lichtträger 2 besteht aus einem Metallröhrchen, in dem Glasfasern aufgenommen sind. Die hier nicht näher bezeichneten Glasfasern dienen somit als Lichtleiter. Diese Lichtübertragung ist an sich aus dem Stand der Technik bekannt, so daß dies bezügliche Einzelheiten nicht naher erläutert oder zeichnerisch dargestellt werden müssen.

An dem der Schraubkappe 13 gegenüberliegenden Ende ist eine Kupplung 9 zu entnehmen, über die ein spatelförmiges Teil 10 an den Handgriff 12 gekuppelt wird, wie das nachfolgend noch naher erläutert wird. Die Kupplung 9 dient als Halterung zum Halten des spatelförmigen Teils 10 am Handgriff 12.

Derartige Kupplungen zwischen spatelförmigem Teil 10 und einem Handgriff 12 sind ebenfalls an sich aus dem Stand der Technik bekannt und müssen daher nicht im einzelnen erläutert werden.

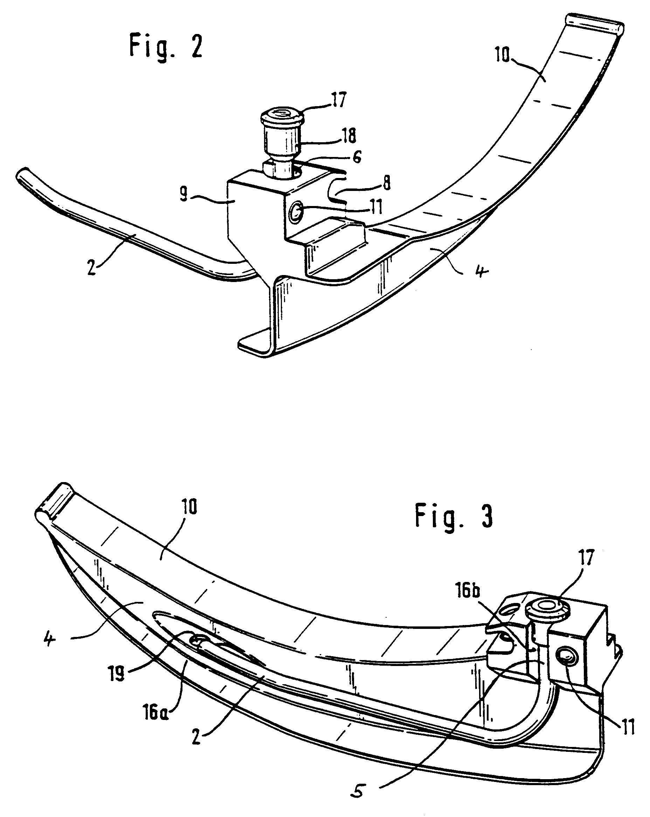

Aus Fig. 2 ist zu entnehmen, daß die Kupplung 9 einen Kupplungshaken 8 aufweist, der in einen, in der Fig. 1 in gestrichelter Linie angedeuteten Stift 20 des Handgriffes 12 eingreift.

In Fig. 2 ist ferner ein seitlicher federbelasteter Kupplungsstift 11 zu entnehmen, der beim Einrasten in den Handgriff 12 in eine entsprechende innere Ausnehmung an einem seitlich in Längsrichtung vorstehenden Abschnitt 22 des Handgriffes 12 eingreift. Auf der gegenüberliegenden Seite der Kupplung 9 ist ein entsprechender weiterer federbelasteter Kupplungsstift vorgesehen, der in eine entsprechende Ausnehmung an einem gegenüberliegenden seitlich vorstehenden Abschnitt des Handgriffes 12 eingreift.

Wie insbesondere aus Fig. 1 zu entnehmen, ist im gekuppelten Zustand der Endbereich der Kupplung 9 zwischen den beiden seitlich vorstehenden Abschnitten 22 des Handgriffes 12 gefangen aufgenommen. Zwischen diesen beiden Abschnitten 22 erstreckt sich der Stift 20, in den der Kupplungshaken 8 eingreift.

Aus Fig. 2 ist ferner zu entnehmen, daß in einem Bereich zwischen dem Kupplungshaken 8 und dem federbelasteten Kupplungsstift 11 eine seitliche Ausnehmung 6 vorgesehen ist, in die ein abgebogener Bereich 5 des Lichtträgers 2 eingreifen kann.

Ferner ist aus der Darstellung von Fig. 1 zu entnehmen, daß, falls das spatelförmige Teil 10 mit dem Handgriff 12 verkuppelt ist, durch die seitlich übergreifenden Abschnitte 22 des Handgriffes 12 ein Verschwenken des Lichtträgers 2 zusätzlich gehindert ist.

Es ist auch möglich, Magnete zur Fixierung des Lichtträgers 2 vorzusehen.

In den Fig. 2 und 3 ist eine Ausführungsform dargestellt, bei der eine mechanische Fixierung des Lichtträgers 2 vorgesehen ist. In Fig. 2 ist dazu eine ausgeschwenkte Position des Lichtträgers 2 dargestellt.

Der Lichtträger 2 ist dazu an seinem der Lichtquelle zugeordneten Ende mit einem Rohrflansch 17 versehen, an den sich ein Gewinde 18 anschließt. Das Gewinde kann auch Teil eines Bajonettverschlusses sein.

Aus Fig. 2 ist zu entnehmen, daß das Ende des Lichtträgers 2, das mit dem Rohrflansch 17 und dem Gewinde 18 versehen ist, in die in Fig. 2 dargestellten verschwenkten Lage so weit angehoben werden kann, bis der Rohrflansch 17 und das Gewinde 18 aus der Ausnehmung 6 herausgetreten sind. Die seitliche Öffnung 16b der Ausnehmung 6 ist dabei so groß, daß nunmehr der Lichtträger 2 seitlich (in der Darstellung von Fig. 2 nach links) abgezogen werden kann. Dementsprechend kann zum Auswechseln ein neuer anderer Lichtträger 2 in umgekehrter Richtung an- bzw. eingesetzt werden. Um den Lichtträger 2 in die in Fig. 3 dargestellte Endstellung 2 zu bringen ist es lediglich notwendig, diesen etwa um 90° zu verschwenken, wobei dieser dann außerdem in eine sich am vorderen Endbereich des spatelförmigen Teils 10 vorhandene seitliche Aufweitung 16a einschmiegt.

In dieser Endstellung ist der Lichtträger 2 entweder eingeklemmt oder mechanisch eingeschraubt, ohne daß zusätzliche Teile, z.B. eine Schraube benötigt wird. Es genügt nämlich die Drehung um die erwähnten etwa 90° des Lichtträgers 2, um das Gewinde 18 in die Ausnehmung in der Kupplung 9 einzuschrauben. Es kann dazu alternativ, wie bereits erwähnt, ein Bajonettverschluß mit einer entsprechenden Bajonettführung vorgesehen sein.

Aus Fig. 3 ist ferner zu entnehmen, daß am vorderen Endbereich des spatelförmigen Teiles 10 eine Öffnung 19 vorgesehen ist, in die das lichtaustrittsseitige Ende des Lichtträgers 2 eingreift.

## Patentansprüche

1. Laryngoskopspatel, insbesondere zum Einführen von Narkosetuben in die Luftröhre,
mit einem Handgriff (12),
mit einem spatelförmigen Teil (10), und
mit einem auswechselbaren Lichträger (2), über den von einer Lichtquelle ausgehende Lichtstrahlen geleitet werden, wobei am spatelförmigen Teil (10) eine Ausnehmung (6) vorgesehen ist, in der zumindest ein Bereich (5) des Lichtträgers (2) lösbar aufnehmbar ist und dort fixierbar ist, wobei der Lichtträger (2) an seinem der Lichtquelle zugeordneten Ende aufgeweitet ist, und wobei die Ausnehmung (6) am spatelförmigen Teil (10) einen aufgeweiteten Bereich aufweist, in dem der aufgeweitete Bereich des Lichtträgers (2) aufnehmbar ist,
dadurch gekennzeichnet, daß der Lichtträger (2) Glasfasern enthält und
daß die Ausnehmung (6) in dem Bereich, in dem der aufgeweitete Bereich des Lichtträgers (2) aufnehmbar ist, eine schmale seitliche Öffnung (16b) aufweist, über die der Lichtträger (2) lediglich in dessen nicht aufgeweitetem Bereich einschiebbar ist und aus dieser ersten Stellung durch eine Verschwenkbewegung in seine Endstellung bringbar ist, wobei in dieser Endstellung der aufgeweitete Bereich des Lichtträgers (2) in den aufgeweiteten Bereich der Ausnehmung (6) eingreift und wegen der schmalen seitlichen Öffnung (16b) vor seitlichem Abfallen gesperrt ist.

2. Laryngoskopspatel nach Anspruch 2, dadurch gekennzeichnet, daß der Lichtträger (2) im Bereich seiner Kupplung mit der Lichtquelle mit einem Rohrflansch (17) versehen ist.

3. Laryngoskopspatel nach Anspruch 1 oder 2, dadurch gekennzeichnet, daß der Lichtträger (2) im aufgeweiteten Bereich mit einer Gewinde- bzw. Bajonettführung versehen ist.

4. Laryngoskopspatel nach einem der Ansprüche 1 bis 3, dadurch gekennzeichnet, daß das spatelförmige Teil (10) mit einer Öffnung (19) versehen ist, durch die das lichtaustrittsseitige Ende des Lichtträgers (2) hindurch geht.

5. Laryngoskopspatel nach einem der Ansprüche 1 bis 4, dadurch gekennzeichnet, daß der Lichtträger (2) zusätzlich über eine Magnetwirkung fixiert ist.

## Claims

1. Laryngoscopic spatula, in particular for introducing anesthetic tubes into the trachea, having
a handle (12),
a spatula-shaped portion (10) and
a replaceable light pipe (2) via which light rays emitted by a light source are guided, wherein said spatula-shaped portion (10) is provided with a recess (6) adapted for separately receiving at least an area (5) of said light pipe (2), said area can be fixed therein, said light pipe (2) is enlarged at its end associated to the light source, and wherein said recess (6) of said spatula-shaped portion (10) is provided with an enlarged section for receiving the enlarged section of said light pipe (2),
characterized in that said light pipe (2) contains glass fibers, and
in that the recess (6) is provided with a narrow lateral opening (16b) in the area for receiving the enlarged section of the light pipe (2), said light pipe (2) can be inserted via said opening (16b) with its not enlarged section only, and said light pipe (2) can be brought from this first position by a pivotal movement to its final position, in which final position the enlarged section of said light pipe (2) engages said enlarged section of said recess (6) and is locked from lateral falling away by the narrow lateral opening (16b).

2. Laryngoscopic spatula according to claim 1, characterized in that the light pipe (2) is provided with a tubular flange (17) in an area of its coupling to the light source.

3. Laryngoscopic spatula according to claim 1 or 2, characterized in that the light pipe (2) is provided in its enlarged section with a threaded guide or a bayonet guide.

4. Laryngoscopic spatula according to anyone of claims 1 through 3, characterized in that the spatula-shaped portion (10) is provided with an opening (19), a light-emitting end of said light pipe (2) passing through said opening (19).

5. Laryngoscopic spatula according to anyone of claims 1 through 4, characterized in that the light pipe is additionally fixed by means of a magnetic effect.

## Revendications

1. Spatule pour laryngoscope, destinée en particulier à l'introduction de tubes à anesthésiques dans la trachée,
comprenant une poignée (12),
comprenant une partie en forme de spatule (10), et
comprenant un support de lumière (2) interchangeable, au moyen duquel des rayons lumineux provenant d'une source lumineuse sont guidés, un évidement (6) étant prévu, sur la partie en forme de spatule (10), dans lequel au moins une zone (5) du support de lumière (2) est susceptible de venir se loger, de façon amovible, et d'y être fixée, le support de lumière (2), à son extrémité associée à la source lumineuse, étant élargi, et l'évidement (6) ménagé dans la partie en forme de spatule (10) présentant une zone élargie dans laquelle la zone élargie du support de lumière (2) est susceptible de venir se loger,
caractérisée en ce que le support de lumière (2) contient des fibres de verre et
en ce que l'évidement (6), dans la zone où vient se loger la zone élargie du support de lumière (2), présente une étroite ouverture latérale (16b), par laquelle le support de lumière (2) peut être inséré, uniquement dans sa zone non élargie et, à partir de cette première position, peut être amené dans sa position terminale, par un mouvement pivotant, la zone élargie du support de lumière (2), dans cette position terminale, pénétrant dans la zone élargie de l'évidement (6) et étant bloqué, sans possibilité de tomber latéralement, du fait de l'étroite ouverture latérale (16b).

2. Spatule de laryngoscope selon la revendication 1, caractérisée en ce que le support de lumière (2), dans la zone de son accouplement avec la source de lumière, est équipé d'une bride tubulaire (17).

3. Spatule de laryngoscope selon la revendication 1 ou 2, caractérisée en ce que le support de lumière (2), dans la zone élargie, est pourvu d'un raccord à vis ou à baïonnette.

4. Spatule de laryngoscope selon l'une des revendications 1 à 3, caractérisée en ce que la partie en forme de spatule (10) est pourvue d'une ouverture (19) à travers laquelle passe l'extrémité du support de lumière (2) qui est située du côté de la sortie de la lumière.

5. Spatule de laryngoscope selon l'une des revendications 1 à 4, caractérisée en ce que le support de lumière (2) est fixé, de façon supplémentaire, par effet magnétique.
